# EUROPÄISCHE PATENTSCHRIFT

(11) **EP 1 289 562 B1**
(45) Veröffentlichungstag und Bekanntmachung des Hinweises auf die Patenterteilung: **29.12.2004**
(21) Anmeldenummer: 01933457.2
(22) Anmeldetag: 21.05.2001
(51) Int. Cl.: A61K 47/48

(54) **PRÄPARATION VON HYPERICIN GEBUNDEN AN POLY-N-VINYLAMIDEN**
NOVEL PREPARATION OF HYPERICIN BONDED WITH POLY-N-VINYLAMIDES
NOUVELLE PREPARATION D'HYPERICINE LIEE A DES POLY-N-VINYLAMIDES

(30) Priorität: 23.05.2000 AT 8962000
(43) Veröffentlichungstag der Anmeldung: 12.03.2003
(73) Patentinhaber: Kubin, Andreas, 1120 Wien (AT); Loew, Hans Günter, 1090 Wien (AT)
(72) Erfinder: Kubin, Andreas, 1120 Wien (AT); Loew, Hans Günter, 1090 Wien (AT)
(86) Internationale Anmeldenummer: PCT/AT2001/000159
(87) Internationale Veröffentlichungsnummer: WO 2001/089576

(56) Entgegenhaltungen:
- EP-A- 0 702 957
- WO-A-00/25824
- WO-A-01/07009
- WO-A-01/85212
- WO-A-01/85213
- DE-A- 19 756 677
- DIWU Z: "Novel therapeutic and diagnostic applications of hypocrellins and hypericins." PHOTOCHEMISTRY AND PHOTOBIOLOGY, (1995 JUN) 61 (6) 529-39. REF: 118 , XP001053406

## Beschreibung

Hypericin kommt in der Natur häufig vor und ist ein Farb- bzw. Pflanzeninhaltsstoff, der nicht nur in *Hypericum perforatum* (Johanniskraut) enthalten ist, sondern auch in vielen änderen Hypericum Species (*Hypericum hirsutum, H. montanum*, etc.) sowie in verschiedenen anderen Pflanzen (z.B. in Buchweizen mit Seitenketten als Fagopyrin). Weiters ist dieses Pigment in Protozoen (*Blepharisma*, *Stentor coeruleus*) zu finden, in Halbflügler (*Wanzenz, Hemiptera*) und als Vorstufe im Blätterpilz *Dermocybe austroveneta*. Die Flechtenart *Nephroma laevigatum* enthält ebenfalls Hypericinderivate.

Über Emodin aus *Cortex fangulae* (Faulbaumrinde) ergibt sich ein einfacher semisynthetischer Weg zu Hypericin.

Hypericin wird seit etwa 20 Jahren auf seine Einsatzmöglichkeiten für therapeutische Anwendungen überprüft. Die lipiden und wasserunlöslichen Eigenschaften von Hypericin erschweren jedoch die Applikation im menschlichen Körper.

EP 0 702 957 B1 und DE 197 56 677 A1 beschreiben jeweils Arzneistoffextrakte aus verschiedenen Pflanzen, die nach Zugabe von Polyvinylpyrrolidon (einem Poly-N-vinylamid) bis zur Trockene eingeengt werden. Es entsteht dabei eine heterogene Mischung aus einigen hundert bis tausend verschiedene Pflanzeninhaltsstoffen und Polyvinylpyrrolidon (im folgenden kurz PVP genannt), die schließlich für peroralen Einnahme Anwendung finden soll.

In den Dokumenten WO 94/14956 und US 5 952 311 B1 wird Hypericin mit einer energiespendenden Substanz wie Luciferin vermischt. Diese chemische Aktivatorsubstanz überträgt schließlich Energie auf den Photosensitizer.

In den letzten 25 Jahren entwickelte sich die selektive Photosensibilisierung von malignen Geweben, oder Gewebeerkrankungen anderer Art zu einem eigenständigen medizinischen Gebiet [1, 2, 6]. Darin finden insbesondere zwei Methoden klinische Anwendung [3]:
- Ausnutzung physikalischer Effekte (z.B. lichtinduzierte Fluoreszenz) zur Diagnose (Photophysikalische Diagnose, PPD)
- Ausnutzung der lichtinduzierten photodynamischen Prozesse als Therapieform (Photodynamische Therapie, PDT)

Die Entwicklung der PDT und PPD zeigt heute einen progressiven Verlauf. Seit 1992 hat sich der monatliche Output an wissenschaftlichen Publikationen nahezu verdreifacht (Current Contents: Life Sciences). Dieses Faktum ist einerseits darauf zurückzuführen, daß sich die PDT als effektive Tumortherapie erwiesen hat und andererseits auch für nichtmaligne Erkrankungen wie z.B. Gefäß-, Viruserkrankungen Anwendung findet.

PDT und PPD bedienen sich Mechanismen, die der Wechselwirkung zwischen Licht und Gewebe zugrundeliegen. Vermittler dieser Wechselwirkungen sind ein geeigneter Photosensitizer und Sauerstoff. So ergeben sich vier Einflußbereiche - Licht, Sensitizer, Sauerstoff und Gewebematrix (physikalisch-optische und chemische Gewebebeschaffenheit [4, 5]), deren Zusammenwirken die zielführende Anwendung von PDT und PPD ermöglicht. Photosensitizern, die geringe systemische Toxizität aufweisen und sich im Idealfall im malignen Tumor anreichern, werden verabreicht. Darauffolgende Belichtung mit sichtbarem Licht induziert photochemische Reaktionen vor allem vom Typ II, aber auch Typ I, die weitgehend eine Zerstörung von Biomembranen, Biomolekülen und subzellulären Organellen hervorrufen [3]. Diese Reaktionen werden therapeutisch in der photodynamischen Tumortherapie genützt, in dem cytotoxische Reaktanden der aktivierten Photosensitizer Tumorzellen zerstören. Weiters können in malignen Läsionen angereicherte Sensitizer über ihr Fluoreszenzlicht zu diagnostischen Methoden etabliert werden.

Die vorliegende Erfindung betrifft nun in einem ersten Aspekt eine Wirkstoffkombination zur Diagnostik und Behandlung von Tumoren, umfassend einen wasserlöslichen Komplex bzw. eine wasserlösliche Verbindung von reinem Hypericin und einem Poly-N-vinylamid. Es ist bekannt, dass Poly-N-vinylamide, wie z.B. PVP, Farbstoffe komplexieren, überraschenderweise stellte sich in Experimenten heraus, daß auch lipophile Farbstoffe wie Hypericin so in wässrige Lösung gebracht werden können. Besonders für die systemische Applikation grösserer Mengen (bis 5 mg / kg Körpergewicht) fehlte bisher die entsprechende wässrige Verabreichungsform, die nun durch die erfindungsgemäße Wirkstoffkombination erstmals zur Verfügung gestellt wird.
Insbesondere betrifft die vorliegende Erfindung eine Wirkstoffkombination umfassend einen wasserlöslichen Komplex bzw. eine wasserlösliche Verbindung von reinem Hypericin und Polyvinylpyrrolidon (PVP) verschiedenen Polymerisations- und Vernetzungsgrades. Durch die Bindung der Reinsubstanz Hypericin an Polyvinylpyrrolidon (PVP) kann ein gut wasserlöslicher Komplex des an sich wasserunlöslichen Sensitizer Hypericin dargestellt werden, der für therapeutische und diagnostische Anwendungen geeignet ist. Durch die Komplexierung von Hypericin an PVP steht erstmals eine wasserlösliche Form des Hypericin zur Verfügung, die leicht applizierbar ist und mit der eine bessere Verteilung des Sensitizers und eine verbesserte Anreicherung und Selektivität in pathologischem Gewebe erreicht werden kann. Die Verwendung von stark gewebeirritierenden organischen Lösungsmitteln wird somit vermeidbar und eine physiologisch günstige Applikationsform von Sensitizern (Hypericin komplexiert mit PVP = Farbstoffkomplex) zur Diagnostik oder Behandlung von malignen aber auch nichtmalignen Erkrankungen darstellbar.

Die wasserlösliche Wirkstoffkombination kann beispielsweise zur Durchführung der photophysikalischen Diagnostik (PPD) eingesetzt werden. Nach selektiver Bindung des Komplexes an Läsionen humanen Gewebes wird z.B mit elektromagnetischer Strahlung (Licht) das Hypericin als Farbstoff angeregt und die physikalische bzw. chemische Response zur Diagnostik genützt. Läsionen z.B. in der Blase (durch Spülung) konnten so angefärbt und über Fluoreszenzlicht nach Anregung erfolgreich detektiert werden.

Die Reinsubstanz Hypericin kann z.B. aus Pflanzen isoliert sein oder es kann auch synthetisches Hypericin, etwa aus Emodin synthetisiert, zum Einsatz kommen, gebunden oder komplexiert an Polyvinylpyrrolidon (PVP) verschiedenen Polymerisations- und Vernetzungsgradesgrades. Dadurch kann der ansonsten wasserunlösliche Farbstoff Hypericin zu verschiedenen medizinischen Zwecken in wässriger Form verabreicht werden. Hypericin wird in chemisch reiner Form eingesetzt, Pflanzenextrakte, die aus einigen hundert Substanzen zusammengesetzt sind, sind nicht geeignet und nicht Gegenstand der vorliegenden Erfindung. PVP finden im Stand der Technik Verwendung als Blutserumersatz, künstliche Tränenflüssigkeit, zur Entgiftung und als Tablettenbinde- und Überzugsmittel (orale Anwendung). Als Blutserumersatz werden heute jedoch überwiegend Dextrane, Gelatine-Stärke-Derivate und Serumproteinlösungen verwendet, die ebenfalls Farbstoffe komplexieren können und in der erfindungsgemäßen Wirkstoffkombination als Hilfsstoffe zum Einsatz kommen können. Durch die Verwendung dieser unterschiedlichen Lösungsvermittler wird auch die Gewebeselektivität und -affinität verändert und es wird somit eine selektivere Anreicherung im entsprechenden Gewebe begünstigt.

Bevorzugt werden Polyvinylpyrrolidone (PVP) mit einem Polymerisationsgrad von niedrigen Molmassen (10.000 - 90.000 g/Mol insbesondere 10.000 - 40.000 g/Mol) verwendet, da diese durch Zellmembranen diffundieren können und ausscheidbar sind. Nach Bedarf kann der Polymerisationsgrad von PVP auch höher festgelegt werden.

Gemäß einer bevorzugten Ausführungsform der vorliegenden Erfindung beträgt in der Wirkstoffkombination das molare Verhältnis von Hypericin zu Poly-N-vinylamid etwa 1:1. Insbesondere liegen in der wasserlöslichen Wirkstoffkombination je nach Anwendungsbereich etwa folgende Konzentrationen vor:
Hypericin von 1 µMol/l bis 0,1 Mol/l
Poly-N-vinylamid (PVP) von 1 µMol/l bis 0,1 Mol/l

Das molare Verhältnis von PVP zu Hypericin ist darüber hinaus abhängig vom Polymerisations- und Vernetzungsgrades des PVP. Je kleiner die molare Masse von PVP, desto höher muss dessen Konzentration im Verhältnis zu Hypericin vorliegen, um das Hypericin als Sensitizer vollständig zu komplexieren. Bei topischer Verabreichung ist auf Grund der geringeren Diffusionsgeschwindigkeit die Konzentration von Hypericin noch höher anzusetzen.

Weiters betrifft die vorliegende Erfindung ein Verfahren zur Herstellung der erfindungsgemäßen Wirkstoffkombination, wobei Hypericin an einem Poly-N-vinylamid, bevorzugt PVP, gebunden beziehungsweise komplexiert wird. Die Komplexierung wird dabei vorzugsweise in wässriger, gegebenenfalls abgepufferter Lösung vorgenommen. So kann beispielsweise Hypericin (aus *Hyperecum perforatum* isoliert, oder z.B. aus Emodin synthetisiert) in wässriger Lösung (etwa physiolog. Kochsalzlösung) mit Polyvinylpyrrolidon (PVP) zur Bildung der erfindungsgemäßen wasserlöslichen Wirkstoffkombination komplexiert werden. Durch das erfindungsgemäße Verfahren werden aktivierbare Komplexe bzw. Verbindungen hergestellt, welche Hypericin umfassen, das an Poly-N-vinylamide verschiedenen Polymerisationsgrades komplexiert oder kovalent gebunden ist.

Vorzugsweise wird die Wirkstoffkombination weiters in an sich bekannter Weise zur intravenösen, intratavitären, inhalativen, oralen, intraperitonealen und topischen Verabreichung, in hydrophilen oder hydrophoben Trägerstoffen, vorzugsweise in Form einer Lösung, einer Creme, eines Gels, eines Aerosols, von Emulsionen oder als Pflaster bereitgestellt.

Gemäß einem weiteren Aspekt der vorliegenden Erfindung ist die Verwendung der erfindungsgemäßen Wirkstoffkombination zur Herstellung eines Arzneimittels vorgesehen, insbesondere zur Herstellung eines Arzneimittels zur Behandlung von Tumoren und krankem Gewebe. Der Behandlung zugänglich sind vor allem Tumore und Läsionen auf und unter der Haut, wie auch in Kavitäten und Lumina oder tiefer liegenden Gewebeteilen. Der Transport der erfindungsgemäßen Wirkstoffkombination erfolgt vorzugsweise über die Gefäße oder durch Diffusion durch Haut und/oder Gewebe zu den Läsionen, wo eine selektive Anreicherung der Wirkstoffkombination aufgrund unterschiedlicher Morphologie und Physiologie beispielsweise von Tumoren stattfindet. Nichtphysiologische (pathologische) Vorgänge im Organismus umfassen sowohl gutartige wie auch bösartige Erkrankungen. All diese Erkrankungen zeichnen sich durch erhöhten Stoffwechsel aus. Auf Grund auch dieser Tatsache lagert sich die erfindungsgemäße Wirkstoffkombination bevorzugt in diesen Körperregionen an. Dadurch ist eine erhöhte Sensibilisierung des erkrankten Gewebeareals zu erzielen. Die zur Anregung erforderliche Lichtenergie (elektromagnetische Strahlung) kann beispielsweise mittels Lichtwellenleiter in das Zielgewebe gebracht werden. In kavitären Regionen (Hohlräumen wie z.B. auch die Blase) kann durch Spülung des Gewebes auch mit höhermolekularem PVP die Diffusion des Farbstoffkomplexes in die Läsionen ermöglicht werden.

Bevorzugt wird auch die Verwendung der erfindungsgemäßen Wirkstoffkombination zur Herstellung eines Diagnostiziermittels zur photophysikalischen beziehungsweise photodynamischen Diagnostik und zur Krebsfrüherkennung.

Die erfindungsgemäße Wirkstoffkombination reichert sich sowohl in malignen als auch in benignen Läsionen an, nicht aber in gesundem Gewebe. Dadurch ist die Detektion von Läsionen über emittiertes Fluoreszenzlicht möglich (Diagnostik). Die selektive Sensibilisierung ermöglicht weiters die therapeutische Behandlung (photodynamische Therapie).

Wenn bei der ersten Behandlung mit der erfindungsgemäßen Wirkstoffkombination nicht die vollständige Entfernung malignen oder pathologischen Gewebes erreicht werden kann, ist eine Wiederholung der Therapie möglich. Es wird gegebenenfalls sodann mehrmals, im Abstand von einigen Wochen nachbehandelt.

Die erfindungsgemäße Wirkstoffkombination wird bei systemischer Anwendung etwa 0,1 bis 36 Stunden vor der Behandlung bzw. Diagnose verabreicht. Dabei erwiesen sich Dosierungen von 0,1 mg bis 5,0 mg Wirkstoffkombination pro kg Körpergewicht als günstig. Die Anreicherung im Zielgewebe wird nach herkömmlichen Methoden (Fluoreszenzspektroskopie) bestimmt.

Die Komplexierung von Hypericin und PVP zeichnet sich durch die besondere Selektivität aus. Die Substanz reichert sich nach ersten Untersuchungen mittels konfokaler Lasermikroskopie in Tumorzellen an und gelangt bis in das endoplasmatische Retikulum der Zellen, nicht aber in den Zellkern (chemisch/toxische Effekte auf die genetische Information im Zellkern können dadurch hintangehalten werden). Die Vorteile gegenüber den erst vereinzelt betriebenen Studien mit anderen Sensitizern wie Porphyrinen und deren Precursoren (5-Aminolävulinsäure) sind:
- Neben Photosensibilisierung des betroffenen Gewebes kommt es kaum zu Nebenwirkungen: Hypericin-PVP ist für den Metabolismus relativ inert,
- kurze biologische Halbwertszeit von Hypericin (34 - 38 h),
- gute Wasserlöslichkeit des Komplexes, jedoch ist Hypericin selbst lipophil und kann aus dem Komplex in lipide Zellkompartimente abgegeben werden,
- die bisherigen Ergebnisse lassen eine ausgezeichnete Tumorselektivität erwarten, mit vielen therapeutischen und diagnostischen Anwendungsmöglichkeiten,
- Hypericin ist ein natürlicher Pflanzeninhaltsstoff, die Rohstoffe sind Johanniskraut, dass in Österreich kultivierbar ist (Porphyrine sind synthetisch bzw. aus Rinder- oder Schweineblut).

Die vorliegende Erfindung wird nun unter Bezugnahme auf die beiliegenden Figuren näher erläutert.

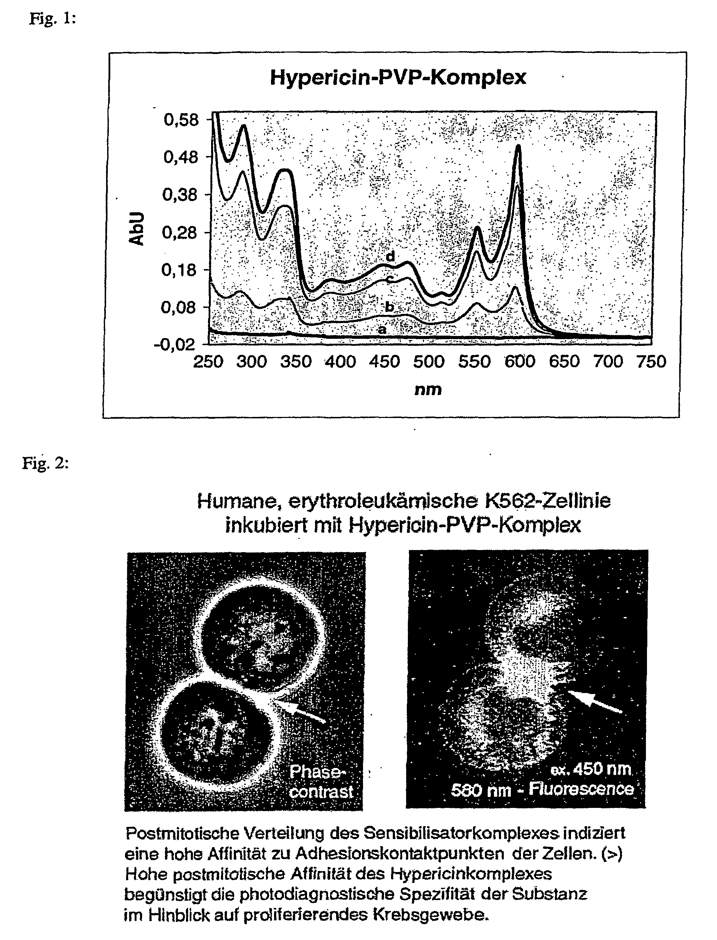

Fig. 1 zeigt die zeitabhängige Löslichkeit von Hypericin nach Komplexierung durch PVP. Ohne PVP zeigt sich keine Absorption im relevanten Spektralbereich (Kurve a), Hypericin bildet in wässriger Umgebung sofort unlösliche Aggregate, die ausfallen und vor allem keine Fluoreszenz mehr zeigen (siehe Diwu Z. et al. [14]). Die vorliegende Lösung von Hypericin-PVP zeigt hingegen das typische Absorbtionsspektrum und emittiert Fluoreszenzlicht bei etwa 600 nm Wellenlänge (Hypericin + PVP nach 30 Minuten, Kurve b; Hypericin + PVP nach 60 Minuten, Kurve c; Hypericin + PVP nach 180 Minuten, Kurve d). Dies zeigt, dass Hypericin in gelöster Form vorliegt (Hypericin in aggregierter oder mikrodispersiver Form emittiert nach Anregung kein Fluoreszenzlicht).

In Fig. 2 werden Mikroaufnahmen von humanen, erythroleukämischen Zellen der K562-Zelllinie gezeigt, welche mit dem erfindungsgemäßen Hypericin-PVP-Komplex inkubiert wurden. Die postmitotische Verteilung des Komplexes legt eine hohe Affinität zu Adhäsionskontaktpunkten der Zellen nahe (siehe Pfeil), wodurch die photodiagnostische Spezifität des erfindungsgemäßen Hypericin-PVP-Komplexes im Hinblick auf proliferierendes Krebsgewebe begünstigt wird.

### Klinische Untersuchung:

Die Methode wurde bei vier Patienten/innen mit gut differenzierten Blasentumoren erprobt. Hier wurde über einen dünnen Blasenkatheter die Substanz (Hypericin-PVP-Lösung) in die Harnblase instilliert. Zwei Stunden später erfolgte dann eine Blasenspiegelung mit Hilfe eines Fluoreszenzsystems (D-Light, Fa. Storz, DE). Hierbei wird ein blauviolettes Licht zur Anregung der Fluoreszenz eingesetzt. Die Fluoreszenzdetektion erfolgt mit Hilfe einer Optik, die ein schmalbandiges Gelbfilter enthält, wodurch die deutliche Rotfluoreszenz mit dem Auge erkannt werden kann. Es zeigte sich eine deutliche Fluoreszenz des sichtbaren Harnblasentumors, der auch unter Weißlichtbetrachtung deutlich sichtbar war. Zusätzlich zeigten sich 2 kleinere, deutlich fluoreszenzpositive Areale, die unter Weißlicht nicht erkannt worden waren. Bemerkenswert ist, dass es unter der längeren Bestrahlung mit dem blauvioletten Licht nicht zu einem Ausbleichen der Fluoreszenz kam.

Ein weiterer Untersuchungsansatz ist die Fluoreszenzzytologie. Bei der Zytologie wird der Harn des Patienten auf maligne Zellen untersucht, um festzustellen, ob der Patient möglicherweise ein Blasentumorrecidiv hat. Die schlecht differenzierten Blasentumoren können in der konventionellen Harnzytologie sehr gut erkannt werden. Leider sind aber die Zellen des gut differenzierten Blasenkarzinoms zytologisch nur schwer von normalen Urothelien zu differenzieren. Da jedoch auch die gut differenzierten Blasentumoren fluoreszenzpositiv sind (in den oben beschriebenen Fällen handelte es sich um gut differenzierte Blasentumore), kann anhand der Fluoreszenz der ausgeschiedenen Zellen das Vorliegen eines Blasentumors diagnostiziert werden.

Neben der präzisen Lokalisation der Tumore kann in Folge auch die therapeutische Intervention durch photodynamische Therapie durchgeführt werden. Der Tumor (beziehungsweise die Läsion), der den Photosensitizer angereichert hat, kann mit entsprechendem Anregungslicht und Energieeintrag auf diese Weise nekrotisiert werden.

### LITERATUR

[1] DOUGHERTY T.J., MARCUS S.L.,
   Photodynamic Therapy.
   Eur J Cancer, 28 (1992) 1734-1742
[2] HILLEGERSBERG R., KORT W.J., WILSON J.H.P.,
   Current Status of Photodynamic Therapy in Oncology.
   Drugs, 48 (1994) 510-527
[3] SROKA R.,
   In-vivo Untersuchung Modifizierter Photosensibilisatoren und Entwicklung von Lichtapplikationssystemen für die PDT.
   Akademischer Verlag München,1992
[4] BHATTA N., ISAACSON K., BHATTA K.M., ANDERSON R.R., SCHIFF I.,
   Comparative study of different laser systems
   Fertility and Sterility, 1994, 61, 4; 581-591
[5] BOCK G., HARNETT S.,
   Photosensitizing Compounds: Their Chemistry, Biology and Clinical Use.
   Ciba Foundation Symposium 146
   John Wiley & Sons, 1989
[6] HENDERSON B.W., DOUGHERTY T.J.
   How does photodynamic therapy work
   Photochem. Photobiol. 55 (1992) 145 - 157
[7] KUBIN A., ALTH G., JINDRA R., JESSNER G., EBERMANN R.
   Wavelength dependent photoresponse of biological and aqueous model systems using the photodynamic plant pigment hypericin.
   J. Photochem. Photobiol. B: Biology 36 (1996) 103 - 108
[8] THOMAS C., PARDINI R. S.
   Oxygen Dependence of Hypericin.induced Phototoxicity to EMT6 Mouse Mammary Carcinoma Cells
   Photochemistry Photobiology, Vol. 55, No. 6, pp. 831-837, 1992
[9] KNOX J.P., DODGE A.D.
   Isolation and activity of the photodynamic pigment hypericin
   Plant, Cell and Environment 8 (1985), 19 - 25
[10] DURAN N., SONG P.S.,
   Hypericin and its Photodynamic action,
   Photochemistry and Photobiology, Yearly Review, Vol. 43, No. 6, pp. 677-680, 1986
[11] VANDENBOGAERDE AL., CUVEELE JF., PROOT P., HIMPENS BE., MERLEVEDE WJ., deWITTE PA.
   Differential cytotoxic effects induced after photosensitization by hypericin.
   J Photochem. Photobiol. B: Biology 38 (1997) 136-142
[12] KNOX JP., DODGE AD.
   Isolation and activity of the photodynamic pigment hypericin
   Plant Cell and Environment 8 (1985) 19-25
[13] ANDREONI A., COLASANTI A., COLASANTI P., MASTROCINQUE M., RICCIO P., ROBERTI G.
   Laser photosensitization of cells by hypericin
   Photochem. Photobiol. 59 (1994) 529-533
[14] DIWU Z.
   Novel therapeutic and diagnostic applications of hypocrellins and hypericins
   Photochem. Photobiol. 61 (1995) 529-539

### Weiterführende Literatur

[15] HESSE M., MEIER H., ZEEH B.,
   Spektroskopische Methoden in der organischen Chemie,
   G. Thieme Vlg. Stuttgart New York 1984, pp. 1 - 32.
[16] ELSTNER E.F.,
   Der Sauerstoff
   Wissenschaftsverlag Mannheim, Wien, Zürich 1990, Band I
[17] YOUNG S.W., QING F., et al.
   Gadolinium(III)texaphyrin: A tumor selective radiation sensitizer that is detectable by MRI.
   Proc. Natl. Acad. Sci. USA, 93 (1996) pp. 6610 - 6615
[18] NITZAN Y, GUTTERMAN M, MALIK Z, EHRENBERG B.
   Inactivation of Gram-negative bacteria by photosensitized porphyrins.
   Photochem. *Photobiol*. *55*, 1992, 89-96
[19] HILL J.S., KAHL S.B., STYLLI S.S., NAKAMURA Y., KOO M.S., KAYE A.H.,
   Selective tumor kill of cerebral glioma by photodynamic therapy using a boronated porphyrin photosensitizer.
   Proc. Natl. Acad. Sci. USA 92 (1995) 12126 - 12130
[20] SZEIMIES R.M., CALZAVARA PINTON PG., KARRER S., ORTEL B., LANDTHALER M.
   Topical photodynamic therapy in dermatology.
   J. Photochem. Photobiol. B: Biology 36 (1996) 213 - 219
[21] PENG Q., WARLOE T., et al.
   Distribution of 5-Aminolevulinic acid-induced Porphyrins in noduloucerative basal cell carcinoma.
   Photochem. Photobiol. 62 (1995) 906 - 913
[22] BRAICHOTTE D., SAVARY JF., GLANZMANN T., WESTERMANN P., FOLLI S., WAGNIERES G., MONNIER P., VAN DEN BERGH H.,
   Clinical pharmacokinetic studies of tetra(meta-hydroxyphenyl)chlorin in squamous cell carcinoma by fluorescence spectroscopy at 2 wavelengths.
   Int. J. Cancer 63 (1995) 198 - 204
[23] SUSLICK KS.
   Die chemische Wirkung von Ultraschall
   Spektrum der Wissenschaften, 4 (1989) 60 - 66

## Patentansprüche

1. Verwendung einer neuen Substanzgruppe umfassend einen wasserlöslichen Komplex bzw. eine wasserlösliche Verbindung von reinem Hypericin und Poly-N-Vinylamid zur Herstellung eines Arznei- oder Diagnostiziermittels zur Diagnostik und Behandlung von Tumoren sowie zur Behandlung von krankem Gewebe.

2. Verwendung nach Anspruch 1, **dadurch gekennzeichnet, dass** zur Diagnostik und Behandlung von Tumoren die neue Substanzgruppe einen wasserlöslichen Komplex bzw. eine wasserlösliche Verbindung von Hypericin (chemisch rein) und Polyvinylpyrrolidon (PVP, als Repräsentant der Gruppe der Poly-N-Vinylamide) verschiedenen Polymerisations- und Vernetzungsgrades umfasst mit Bezeichnung "PVP-Hypericin".

3. Verwendung nach Anspruch 2, **dadurch gekennzeichnet, dass** Polyvinylpyrrolidon einen Polymerisationsgrad von niedrigen Molmassen aufweist, vorzugsweise von 10.000-90.000 g/Mol, insbesondere bevorzugt von 10.000-40.000 g/Mol.

4. Verwendung nach einem der Ansprüche 1 bis 3 , **dadurch gekennzeichnet, dass** in der neuen Substanzgruppe das molare Verhältnis von Hypericin zu Poly-N-vinylamid etwa 1:1 beträgt.

5. Verwendung nach einem der Ansprüche 1 bis 4, **dadurch gekennzeichnet, dass** in der neuen Substanzgruppe der Konzentrationsbereich an Hypericin und an Poly-N-Vinylamid (z.B PVP) jeweils von 1 mikroMol /l bis 0,1 Mol/l beträgt.

6. Verwendung nach einem der Ansprüche 1 bis 5, **dadurch gekennzeichnet, dass** bei der Herstellung der neuen Substanzgruppe Hypericin an einem Poly-N-vinylamid, bevorzugt PVP, gebunden beziehungsweise komplexiert wird.

7. Verwendung nach Anspruch 6, **dadurch gekennzeichnet, dass** die Komplexierung in wässriger, gegebenenfalls abgepufferter Lösung vorgenommen wird.

8. Verwendung nach einem der Ansprüche 6 oder 7, **dadurch gekennzeichnet, dass** die neue Substanzgruppe weiters in an sich bekannter Weise zur intravenösen, intrakavitären, inhalativen, oralen, intraperitonealen und topischen Verabreichung, in hydrophilen oder hydrophoben Trägerstoffen, vorzugsweise in Form einer Lösung, einer Creme, eines Gels, eines Aerosols, von Emulsionen oder als Pflaster oder Sprühverband bereitgestellt wird.

9. Verwendung nach einem der Ansprüche 1 bis 8, **dadurch gekennzeichnet, dass** ein Diagnostiziermittel zur photophysikalischen beziehungsweise photodynamischen Diagnostik und zur Krebsfrüherkennung hergestellt wird.

## Claims

1. Application of a new set of substances comprising an active substance combination of water-soluble complex or water-soluble compound of pure hypericin and a poly-N-vinylamide for preparation as a medicament or diagnostic pharmaceutical for detecting tumors and therapeutically treating diseased tissue in a patient.

2. Application as set forth in claim 1, wherein said poly-N-vinylamide is a polyvinyl pyrrolidone (PVP) with varying polymerization and cross-linking degrees and wherein said hypericin (chemically purified) forms complexes with PVP and said active substance combination is designated as "PVP-Hypericin" and used for diagnosing and treating tumors.

3. Application as set forth in claim 2, wherein said polyvinylpyrrolidone has polymerization degrees corresponding to molar masses ranging from 10000-90000 g /Mol with a preferred range of 10000-40000 g/Mol.

4. Application as set forth in claims 1 - 3, wherein said new set of substances comprises said hypericin and said poly-N-vinylamide at molar ratio of hypericin to poly-N-vinylamide of approximately 1:1.

5. Application as set forth in claims 1 - 4, wherein said hypericin and said poly-N-vinylamide (e.g. PVP) are contained in said active substance combination at a concentration ranging from 1 micromol /l to 0,1 mol /l each.

6. Application as set forth in claims 1 - 5, wherein said hypericin is bound to or complexed with said poly-N-vinylamide ( preferrrably PVP ) to yield said active substance.

7. Application as set forth in claim 6, wherein said complexation reaction of hypericin with poly-N-vinylamide is effected in aqueous solution or buffered aqueous solution.

8. Application as set forth in claim 6 or 7, wherein said new set of active subtances is provided in hydrophilic or in hydrophobic carrier substances, preferrably as solution, or crème, or gel, or aerosol, or emulsion, or plaster, or spraydressing to be administered to said patient in the usual way either intravenously, intracavitaryly, inhalationally, orally, intraperitoneally or topically.

9. Application as set forth in claim 1 - 8, wherein said diagnosing is selected from the group consisting of photophysical and photodynamic diagnosing and wherein said tumor is a cancer, said diagnosing being an early diagnosing of cancer.

## Revendications

1. Utilisation d'un nouveau groupe de substances comprenant un complexe ou un composé soluble dans l'eau d'hypéricine et de poly-N-vinylamide pour la fabrication d'un médicament ou agent diagnostique pour le diagnostic et le traitement de tumeurs et pour le traitement de tissus malades.

2. Utilisation conformément à la revendication 1, **caractérisée par le fait que**, pour le diagnostic et le traitement de tumeurs, le nouveau groupe de substances comprend un complexe ou un combiné soluble dans l'eau d'hypéricine (chimiquement pur) et de polyvinylpyrrolidone (PVP en tant que représentant du groupe des poly-N-vinylamides) d'un degré différent de polymérisation et de réticulation, sous la désignation « PVP-hypéricine ».

3. Utilisation conformément à la revendication 2, **caractérisée par le fait que** le polyvinylpyrrolidone présente un degré de polymérisation de faibles masses molaires, de préférence de 10.000-90.000 g/mol, avec préférence particulière de 10.000-40.000 g/mol.

4. Utilisation conformément à l'une des revendications 1 à 3, **caractérisée par le fait que**, dans le nouveau groupe de substances, le rapport molaire de l'hypéricine et du poly-N-vinylamide est d'environ 1 :1.

5. Utilisation conformément à l'une des revendications 1 à 4, **caractérisée par le fait que**, dans le nouveau groupe de substances, la concentration d'hypéricine et du poly-N-vinylamide (par example PVP) est comprise entre 1 micromol /l et 0,1 mol/l.

6. Utilisation conformément à l'une des revendications 1 à 5, **caractérisée par le fait que**, dans la fabrication du nouveau groupe de substances, l'hypéricine est liée ou complexée à un poly-N-vinylamide, de préférence PVP.

7. Utilisation conformément à la revendication 6, **caractérisée par le fait que** la formation du complexe est effectuée dans une solution aqueuse, éventuellement tamponnèe.

8. Utilisation conformément à l'une des revendications 6 ou 7, **caractérisée par le fait que** le nouveau groupe de substances est mis à la disposition d'une manière connue pour une administration intraveineuse, intracavitaire, inhalée, orale, intrapéritonale et topique dans des supports hydrophiles ou hydrophobes, de préférence sous forme de solution, de crème, de gel, d'aérosol, d'émulsions, d'émplâtre ou de pansements liquides.

9. Utilisation conformément à l'une des revendications 1 ou 8, **caractérisée par le fait qu'**un produit diagnostique est fabriqué pour le diagnostic photophysique, et / ou photodynamique, et pour la détection précoce du cancer.
